Europäisches Patentamt

European Patent Office

Office européen des brevets

(11) Publication number: 0 282 940
A2

# (12) EUROPEAN PATENT APPLICATION

(21) Application number: 88103977.0

(22) Date of filing: 14.03.88

(51) Int. Cl.4: C07C 45/51 , C07C 45/62 ,
C07C 47/24 , C07C 47/263 ,
C07C 47/21 , C07C 41/48

(30) Priority: 16.03.87 JP 60783/87

(43) Date of publication of application:
21.09.88 Bulletin 88/38

(84) Designated Contracting States:
DE FR GB

(71) Applicant: Shin-Etsu Chemical Co., Ltd.
6-1, Ohtemachi 2-chome
Chiyoda-ku Tokyo 100(JP)

(72) Inventor: Fukumoto, Takehiko
Shin-Etsu Kagaku Kirinoki Ryo 27-20,
Sanaicho
Joetsu-shi Niigata-ken(JP)       .
Inventor: Yamamoto, Akira
14-45, Gochi 2-chome
Joetsu-shi Niigata-ken(JP)

(74) Representative: Raeck, Wilfrid, Dipl.-Ing.
Moserstrasse 8
D-7000 Stuttgart 1(DE)

(54) A method for the preparation of a gamma-substituted (E)-2-alken-1-AL.

(57) An efficient method is disclosed for the preparation of an $\omega$-substituted (E)-2-alken-1-al, e.g., 12-chloro-(E)-2-dodecen-1-al in three steps of reactions. The starting compound is an $\omega$-substituted 1-alkynyl magnesium halide which is reacted with trimethyl or triethyl orthoformate to give an $\omega$-substituted 1,1-dialkoxy-2-alkyne followed by a partial hydrogenation of the triple bond and then hydrolysis or, alternatively, followed by the hydrolysis reaction and then partial hydrogenation of the triple bond.

EP 0 282 940 A2

# A METHOD FOR THE PREPARATION OF AN ω-SUBSTITUTED (E)-2-ALKEN-1-AL

The present invention relates to a method for the preparation of an ω-substituted (E)-2-alken-1-al or, more particularly, to a method for the preparation of an ω-substituted (E)-2-alken-1-al useful as an intermediate for the synthetic preparations of various kinds of biologically active compounds such as agricultural chemicals, sex pheromones of insects, growth controlling agents of plants, synthetic perfumes and the like.

ω-Substituted (E)-2-alken-1-als are compounds very useful as an intermediate for the synthetic preparation of the above mentioned biologically active compounds by virtue of their unique characteristics that, in addition to the E-configuration of the geometric structure at the double bond, the substituent group at the ω-position remains intact even after the compound has been subjected to a specific reaction on the aldehyde group, such as the Wittig reaction, leaving a possibility of pertaining to a further reaction to give useful compounds. Several methods are known in the prior art for the synthetic preparation of the compounds of this class but none of them is economically feasible because of the complicated unit processes involved in the method. In one of the known methods, for example, an $\alpha,\beta$-unsaturated carboxylic acid as the starting material is first esterified and the substituent group at the ω-position is protected followed by reduction of the carboxyl group into an alcoholic hydroxy group which is then oxidized into an aldehyde group.

An alternative method is disclosed by Henrick et al. in Tetrahedron, volume 33, page 1845 (1977) for the preparation of 9-tetrahydropyranyloxy-(E)-2-nonen-1-al as an intermediate for the synthesis of (E,Z)-7,9-dodecadienyl acetate. The starting material of the method is 1-bromo-7-tetrahydropyranyloxy heptane and the method involves several specific reaction by use of very specific and expensive reagents rendering the method almost impractical.

Accordingly, it is eagerly desired to develop an economically advantageous method for the synthetic preparation of an ω-substituted (E)-2-alken-1-al.

The present invention, which has been completed as a result of the extensive investigations undertaken with an object to solve the above mentioned problem, provides a novel method for the synthetic preparation of an ω-substituted (E)-2-alken-1-al represented by the general formula

$$OHC\text{-}CH^{(E)}=CH(CH_2)_nR,$$

in which n is a positive integer not exceeding 20 and R is a substituent group, which comprises the steps of:

(1) reacting an ω-substituted 1-alkynyl magnesium halide represented by the general formula

$$XMgC{\equiv}C(CH_2)_nR^1,$$

in which X is a halogen atom, $R^1$ is a substituent atom or group selected from the class consisting of halogen atoms, vinyl group and hydroxy group protected with an organic group or an atom of metal and n is a positive integer not exceeding 20, with a trialkyl orthoformate represented by the general formula

$$(R^2O)_3CH,$$

in which $R^2$ is a methyl or an ethyl group, to form an ω-substituted 1,1-dialkoxy-2 alkyne represented by the general formula

$$(R^2O)_2CH\text{-}C{\equiv}C(CH_2)_nR^1;$$

and

(2) partially hydrogenating the ω-substituted 1,1-dialkoxy-2-alkyne followed by the hydrolysis of the hydrogenation product; or (2') hydrolyzing the ω-substituted 1,1-dialkoxy-2-alkyne followed by the partial hydrogenation of the hydrolysis product.

The starting material in the inventive method is an ω-substituted 1-alkynyl magnesium halide represented by the general formula

$$XMgC{\equiv}C(CH_2)_nR^1,$$

in which X is a halogen atom, $R^1$ is a substituent atom or group selected from the class consisting of halogen atoms, vinyl group and hydroxy group protected with an organic group or an atom of metal and n is a positive integer not exceeding 20. More particularly, the halogen atom denoted by X can be an atom of chlorine, bromine or iodine. The organic group protecting a hydroxy group is exemplified by substituted or unsubstituted silyl groups, e.g., trimethylsilyl, tert-butyl dimethyl silyl, methyl di-tert-butyl silyl, isopropyl dimethyl silyl, phenyldimethylcarbinyl dimethyl silyl and tert-butyl diphenyl silyl groups, substituted or unsubstituted alkyl groups, e.g., methoxy methyl, methylthio methyl, benzyloxy meth-yl, 2-methoxyethoxy meth-yl, 1-ethoxy ethyl, 1-methyl-1-methoxy ethyl, n-butyl, tert-butyl, benzyl and 4-methoxy benzyl groups, and residual groups of heterocyclic compounds, e.g., tetrahydropyranyl and tetrahydrofuranyl groups.

When the substituent denoted by $R^1$ in the ω-substituted (E)-2-alken-1-al is a vinyl group, the compound can be converted into various kinds of other compounds having a different functional group by the reaction of hydroalumination, hydromagnesation, hydroboration and the like at the vinyl group. ω-Substituted (E)-2-alken-1-als having a substituent other than vinyl group also have a possibility of converting the substituent into other functional groups such as halogen, alkanol, aldehyde, acetate, carboxylic acid, ester of carboxylic acid and the like.

Examples of the starting compound represented by the general formula $XMgC \equiv C(CH_2)_nR^1$ include: 5-chloro-1-pentynyl magnesium chloride; 6-chloro-1-hexynyl magnesium chloride; 6-bromo-1-hexynyl magnesium bromide; 8-chloro-1-octynyl magnesium chloride; 8-chloro-1-octynyl magnesium bromide; 8-chloro-1-octynyl magnesium iodide; 8-bromo-1-octynyl magnesium chloride; 8-iodo-1-octynyl magnesium bromide; 11-chloro-1-undecynyl magnesium chloride; 12-chloro-1-dodecynyl magnesium chloride; 18-chloro-1-octadecynyl magnesium chloride; 6-vinyl-1-hexynyl magnesium chloride, i.e. octa-7-en-1-yne magnesium chloride; 9-vinyl-1-nonynyl magnesium chloride, i.e. undeca-10-en-1-yne-1 magnesium chloride; 5-(tert-butyl dimethyl silyloxy)-1-pentynyl magnesium chloride; 8-trimethylsilyloxy-1-octynyl magnesium chloride; 8-(tert-butyl dimethyl silyloxy)-1-octynyl magnesium bromide; 8-(phenyl dimethylcarbinyl dimethyl silyloxy)-1-octynyl magnesium chloride; 6-(1-ethoxy ethyloxy)-1-hexynyl magnesium chloride; 6-(1-methyl-1-methoxy ethyloxy)-1-hexynyl magnesium chloride; 6-tert-butoxy-1-hexynyl magnesium bro-mide; 6-benzyloxy-1-hexynyl magnesium bromide; 8-methoxy-methyloxy-1-octynyl magnesium bromide; 8-benzyloxymethyl-oxy-1-octynyl magnesium chloride; 3-tetrahydropyranyloxy-1-propynyl magnesium chloride; 8-tetrahydropyranyloxy-1-octynyl magnesium chloride; 8-chloromagnesiumoxy-1-octynyl magnesium chloride and the like.

The above described magnesium halide is first reacted with a trialkyl orthoformate which may be trimethyl orthoformate or triethyl orthoformate.

The reaction of the Grignard reagent and the trialkyl orthoformate is undertaken in an organic solvent selected from the group consisting of tetrahydrofuran, benzene, toluene, cumene, diethyl ether, n-butyl ether and the like. A particularly preferable solvent is tetrahydrofuran or a mixture of tetrahydro-furan with other solvents. The reaction is performed at a temperature in the range from 50 to 120 °C or, preferably, from 80 to 100 °C. When the reaction temperature is too low, the velocity of the reaction is unpracticably low. When the reaction temperature is too high, a polymerization reaction may take place to decrease the yield of the reaction product. The reaction is complete usually within 10 to 30 hours. The trialkyl orthoformate is used in an amount of 1.0 to 2.0 moles or, preferably, 1.0 to 1.5 moles per mole of the magnesium halide compound, i.e. the Grignard reagent. The reaction is performed in an atmosphere of an inert gas such as nitrogen.

After completion of the above described reaction, the reaction mixture is admixed with water to decompose the Grignard reagent and the reaction product, i.e. $\omega$-substituted 1,1-dialkoxy-2-alkyne, can be isolated from the reaction mixture by a conventional procedure of isolation such as distillation and column chromatography. The yield of the desired product is usually in the range from 50% to 80% of the theoretical value.

The thus obtained $\omega$-substituted 1,1-dialkoxy-2-alkyne is then partially hydrogenated followed by hydrolysis or hydrolyzed followed by partial hydrogenation to easily give the desired final product of $\omega$-substituted (E)-2-alken-1-al in a good yield.

In the former process of the partial hydrogenation preceding the hydrolysis, the catalyst suitable for promoting the reaction is selected from a P-2 nickel catalyst, Raney nickel catalyst, Lindlar catalyst, palladium catalysts supported on carbon, barium sulphate, alumina, diatomaceous earth, barium carbonate, calcium carbonate and the like, rhodium catalyst supported on carbon, ruthenium catalyst supported on carbon and the like, of which the P-2 nickel catalyst is preferred in respect of inexpensiveness and easiness in preparation. The catalyst is used in an amount of 0.001 to 0.2 equivalent based on the reactants. The pressure of hydrogen in the hydrogenation reaction is in the range from normal pressure to 10 kg/cm$^2$G or, preferably, from 1 to 5 kg/cm$^2$G. The reaction temperature, which greatly influences the reaction velocity, is in the range from 0 to 80 °C or, preferably, in the range from 20 to 50 °C. When the reaction temperature is too high, certain undesirable side reactions may take place to produce undesired products as impurities. After completion of the hydrogenation reaction, the reaction mixture is freed from the solid catalyst by a conventional solid-liquid separation process and then subjected to the hydrolysis reaction.

The reaction of hydrolysis of the partial hydrogenation product is performed by dissolving the hydrogenation product in an organic solvent and admixing the solution with an acid such as hydrochloric acid, formic acid, acetic acid and the like. A preferable acid is hydrochloric acid in a concentration of about 10 to 30%. Suitable organic solvents include hydrocarbon solvents such as benzene, toluene, hexane and the like, tetrahydrofuran and methylene chloride, of which hexane and methylene chloride are preferred. A preferable formulation of the reaction mixture is that the hydrogenation product, 20 % hydrochloric acid and organic solvent are taken and mixed together each in a volume approximately equal to the others. The hydrolysis reaction is performed at a temperature in the range from 0 to 60 °C or, preferably, from 0 to 40 °C. When the temperature is too high, a side reaction of polymerization may take place to form a trimer and other higher polymers. The hydrolysis reaction is complete within 5 to 120 minutes or, usually, within 5 to 60 minutes. After completion of the hydrolysis reaction, the reaction mixture is subjected to phase separation and the organic solution is washed with water repeatedly until neutrality and then freed from the organic solvent so that the desired final product of the ω-substituted (E)-2-alken-1-al is obtained in a considerably high purity. If desired, the thus obtained reaction product can be further purified by a conventional purification procedure such as distillation and column chromatography.

As is mentioned before, the reaction product of the Grignard reagent with the trialkyl orthoformate may be first hydrolyzed prior to the partial hydrogenation reaction. The hydrolysis reaction in this case can be performed in substantially the same manner as in the above described procedure of the hydrolysis reaction succeeding the hydrogenation reaction. Different from the above described hydrogenation reaction preceding the hydrolysis, on the other hand, a preferred catalyst for the partial hydrogenation of the hydrolysis product is a palladium catalyst such as those supported on carbon, alumina and the like, of which carbon-supported palladium catalysts are particularly preferred. The partial hydrogenation reaction is carried out at a temperature in the range from 0 to 50 °C under a hydrogen pressure in the range from normal pressure to 10 kg/cm²G. The partial hydrogenation reaction is performed usually by diluting the hydrolysis product with a suitable organic solvent which is preferably ethyl alcohol, although the reaction can proceed even without using any organic solvents.

Irrespective of the sequential order of the partial hydrogenation reaction and the hydrolysis reaction, the hydrogenation reaction is specific to produce a steric structure of almost complete E-configuration relative to the double bond in the ω-substituted 2-alken-1-al as the desired final product.

When the substituent denoted by $R^1$ in the starting ω-substituted 1-alkynyl magnesium halide of the formula $XMgC{\equiv}C(CH_2)_nR^1$ is a hydroxy group protected by a silyl group other than tert-butyl dimethyl silyl, methyl di-tert-butyl silyl and phenyldimethylcarbinyl dimethyl silyl groups, 1-ethoxy ethyl group, 1-methyl-1-ethoxy-ethyl group, tetrahydropyranyl group or tetrahydrofuranyl group, the substituent group in the final product of ω-substituted (E)-2-alken-1-al has been converted into a hydroxy group in the course of the above described reactions. When the substituent denoted by $R^1$ in the starting compound is a halogen atom or an organic group other than the above mentioned, the substituent in the final product is the same as in the starting compound without being decomposed in the reaction.

In the following, the method of the present invention is described in more detail by way of examples which in no way limit the scope of the present invention.

Example 1.

12-Chloro-(E)-2-dodecen-1-al was synthesized in the following manner.

Thus, a solution of 1 mole of 11-chloro-1-undecynyl magnesium chloride in 200 g of thtrehydrofuran was heated and kept at 70 to 80 °C under a stream of nitrogen gas and 177.6 g (1.2 moles) of triethyl orthoformate were added thereto dropwise. The reaction mixture was agitated at 80 to 100 °C for further 20 hours to effect the reaction between the Grignard reagent and the triethyl orthoformate. After completion of the reaction, the reaction mixture was poured into 400 ml of a 10% aqueous solution of ammonium chloride and the organic solution taken by phase separation was first freed from tetrahydrofuran and then distilled under reduced pressure to give 213 g of a fraction boiling at 160 to 165 °C under a pressure of 2mmHg. This product could be identified to be 12-chloro-1,1-diethoxy-2-dodecyne. The above mentioned yield of the product was about 74% of the theoretical value.

Separately, a P-2 nickel catalyst solution was prepared by dissolving 2.5 g of nickel acetate in 200 ml of ethyl alcohol and then adding 0.4 g of sodium borohydride to the solution followed by agitation for several minutes. The thus prepared catalyst solution was introduced into an autoclave together with 0.5 g of ethylene diamine and 213 g of the above obtained 12-chloro-1,1-diethoxy-2-dodecyne and the autoclave was pressurized up to a pressure of 5 kg/cm²G by introducing hydrogen gas to effect the hydrogenation reaction for 1 hour at 30 to 40 °C.

After completion of the partial hydrogenation reaction, the reaction mixture taken out of the autoclave was admixed with each 200 ml of water and n-hexane and then subjected to phase separation. The organic solution taken by phase separation was admixed with 200 ml of a 20 % hydrochloric acid and agitated for 30 minutes at 30 to 40 °C to effect the hydrolysis reaction. Thereafter, the reaction mixture was subjected to phase separation. The organic solution taken by phase separation was washed with water repeatedly until neutrality and then freed from the organic solvent to give 146 g of a product which could be identified to be 12-chloro-(E)-2-dodecen-1-al. The above mentioned yield of the product was about 91% of the theoretical value based on the amount of 12-chloro-1,1-diethoxy-2-dodecyne.

Example 2.

9-Hydroxy-(E)-2-nonen-1-al was synthesized in the following manner.

Thus, a solution of 1 mole of 8-thtrahydropyranyloxy-1-octynyl magnesium chloride in 200 g of tetrahydrofuran was heated and kept at 70 to 80 °C under a stream of nitrogen gas and 177.6 g (1.2 moles) of triethyl orthoformate were added thereto dropwise. The reaction mixture was heated at 80 to 100 °C for further 20 hours to effect the reaction between the Grignard reagent and the triethyl orthoformate. After completion of the reaction, the reaction mixture was poured into 400 ml of a 10% aqueous solution of ammonium chloride and then subjected to phase separation. The organic solution taken by phase separation was freed from tetrahydrofuran and then subjected to alumina-activated column chromatography to give 184 g of a fraction which could be identified to be 9-tetrahydropyranyloxy-1,1-diethoxy-2-nonyne. The above mentioned yield of the product was about 75% of the theoretical value.

The thus obtained 9-tetrahydropyranyloxy-1,1-diethoxy-2-nonyne was used as the starting material in the successive partial hydrogenation and hydrolysis reactions carried out in the same manner as in Example 1 to give 105 g of a product which could be identified to be 9-hydroxy-(E)-2-nonen-1-al. The yield was about 89.5 % of the theoretical value.

Example 3

5-(tert-Butyl dimethyl silyloxy)-(E)-2-penten-1-al was synthesized in the following manner.

Thus, 194 g of 5-(tert-butyl dimethyl silyloxy)-1,1-diethoxy-2-pentyne were obtained in substantially the same manner as in the preparation of 9-tetrahydropyranyloxy-1,1-diethoxy-2-nonyne in Example 2 excepting the use of a solution of 1 mole of 5-(tert-butyl dimethyl silyloxy)-1-pentynyl magnesium chloride in 250 g of tetrahydrofuran as the starting material. The yield was about 68% of the theoretical value.

The acetal compound obtained in the above mentioned reaction was admixed with 300 g of methylene chloride and 300 g of a 15% hydrochloric acid and agitated for 2 hours at 25 to 35 °C to effect the hydrolysis reaction. At the end of the reaction time for 2 hours, the gas chromatographic analysis of the reaction mixture indicated that the acetal compound has been completely decomposed. Thereafter, the reaction mixture was subjected to phase separation and the organic solution was washed successively first with 300 ml of water and then with 300 ml of a 5% aqueous solution of sodium hydrogencarbonate. The solution was then freed from methylene chloride in a rotary evaporator and the concentrated solution was introduced into an autoclave together with 7 g of a carbon-supported catalyst containing 5% of palladium followed by pressurization of the autoclave up to a pressure of 2 kg/cm²G by introducing hydrogen gas to effect the partial hydrogenation reaction for 1 hour at 10 to 20 °C. After completion of the hydrogenation reaction, the reaction mixture taken out of the autoclave was freed from the catalyst by filtration to give 113 g of a product which could be identified to be 5-(tert-butyl dimethyl silyloxy)-(E)-2-penten-1-al. The yield was about 89% of the theoretical value.

Example 4

11,(E)-2-Dodecadien-1-al was synthesized in the following manner.

Thus, 167 g of 1,1-diethoxydodeca-11-en-2-yne were obtained in substantially the same manner as in the preparation of 12-chloro-1,1-diethoxy-2-dodecyne in Example 1 excepting the use of a solution of 1 mole of undeca-10-en-1-yne 1-magnesium chloride, i.e. 9-vinyl-1-nonynyl magnesium chloride, in 200 g of tetrahydrofuran as the starting compound. The yield of the compound was about 63% of the theoretical value.

The thus obtained acetal compound was subjected to the hydrolysis and partial hydrogenation reactions in substantially the same manner as in the preceding example to give 107 g of a product which could be identified to be 11,(E)-2-dodecadien-1-al. The yield of the product was about 88% of the theoretical value.

Example 5.

9-Chloro-(E)-2-nonen-1-al was synthesized in the following manner.

Thus, 1 mole of 8-chloro-1-octynyl magnesium chloride as a solution in 200 g of tetrahydrofuran was used as the starting material and reacted with triethyl orthoformate in substantially the same manner as in Example 1. The reaction mixture after completion of the reaction was first freed from tetrahydrofuran and then distilled under reduced pressure to give 180 g of a fraction boiling at 155 to 160 °C under a pressure of 5 mmHg, which could be identified to be 9-chloro-1,1-diethoxy-2-nonyne. The yield of the product was about 73% of the theoretical value.

The thus obtained acetal compound was subjected to the partial hydrogenation and hydrolysis reactions in substantially the same manner as in Example 1 to give 118 g of a product which could be identified to be 9-chloro-(E)-2-nonen-1-al. The yield of this final product was about 93% of the theoretical value.

This aldehyde compound could be readily converted by utilizing the Wittig reaction into (E,Z)-7,9-dodecadienyl acetate which is a known sex pheromone of notorious European grape vine moth, i.e. <u>Lobesia botrana</u>.

## Claims

1. A method for the preparation of an $\omega$-substituted (E)-2-alken-1-al represented by the general formula

$$OHC-CH^{(E)} = CH(CH_2)_nR,$$

in which R is a substituent selected from the group consisting of halogen atoms, hydroxy group and vinyl group and n is an integer not exceeding 20, which comprises the successive steps of:

(a) reacting an $\omega$-substituted 1-alkynyl magnesium halide represented by the general formula

$$XMgC \equiv C(CH_2)_nR^1,$$

in which X is a halogen atom, $R^1$ is an atom or group selected from the group consisting of halogen atoms, vinyl group and hydroxy group protected with an organic group or an atom of metal and n has the same meaning as defined above, with trimethyl orthoformate or triethyl orthoformate to form an $\omega$-substituted 1,1-dialkoxy-2-alkyne represented by the general formula

$$(R^2O)_2CH-C \equiv C-CH(CH_2)_nR^1,$$

in which $R^2$ is a methyl or an ethyl group and $R^1$ and n each have the same meaning as defined above;

(b) partially hydrogenating the $\omega$-substituted 1,1-dialkoxy-2-alkyne; and

(c) hydrolyzing the hydrogenation product obtained in step (b).

2. A method for the preparation of an $\omega$-substituted (E)-2-alken-1-al represented by the general formula

$$OHC-CH^{(E)} = CH(CH_2)_nR,$$

in which R is a substituent selected from the group consisting of halogen atoms, hydroxy group and vinyl group and n is an integer not exceeding 20, which comprises the successive steps of:

(a) reacting an $\omega$-substituted 1-alkynyl magnesium halide represented by the general formula

$$XMgC \equiv C(CH_2)_nR^1,$$

in which X is a halogen atom, $R^1$ is an atom or group selected from the group consisting of halogen atoms, vinyl group and hydroxy group protected with an organic group or an atom of metal and n has the same meaning as defined above, with trimethyl orthoformate or triethyl orthoformate to form an $\omega$-substituted 1,1-dialkoxy-2-alkyne represented by the general formula

$$(R^2O)_2CH-C \equiv C(CH_2)_nR^1,$$

in which $R^2$ is a methyl or an ethyl group and $R^1$ and n each have the same meaning as defined above;

(b) hydrolyzing the $\omega$-substituted 1,1-dialkoxy-2-alkyne; and

(c) partially hydrogenating the hydrolysis product obtained in step (b).

3. The method for the preparation of an $\omega$-substituted (E)-2-alken-1-al as claimed in claim 1 wherein the hydrogenation reaction in step (b) is performed in the presence of a P-2 nickel catalyst.

4. The method for the preparation of an $\omega$-substituted (E)-2-alken-1-al as claimed in claim 2 wherein the hydrogenation reaction in step (c) is performed in the presence of a palladium catalyst.